# EUROPEAN PATENT SPECIFICATION

(11) **EP 3 175 865 B1**
(45) Date of publication and mention of the grant of the patent: **15.04.2026**
(21) Application number: 15771713.3
(22) Date of filing: 31.07.2015
(51) Int. Cl.: A01P 1/00, A01N 37/46, A01N 47/44, A01N 33/12, A01N 25/26, A61K 8/25, A61K 8/27, A61K 8/58, A61Q 17/02, A61Q 17/04, A61Q 19/00, A61K 9/00, A61K 9/16, C01B 33/12, A61L 15/44, A61L 15/46, C09D 5/00, C11D 3/08, D06M 11/46, C09D 7/40

(54) **FUNCTIONAL PARTICLES, PROCESS TO OBTAIN THEM, AND THEIR APPLICATIONS**
FUNKTIONELLE PARTIKEL, HERSTELLUNGSVERFAHREN UND VERWENDUNGEN
PARTICULES FONCTIONNELLES, PROCÉDÉ D'OBTENTION ET APPLICATIONS CORRESPONDANTES

(30) Priority: 01.08.2014 PT 10782814
(43) Date of publication of application: 07.06.2017
(73) Proprietor: Smart Inovation Lda, 4755-539 Barcelos (PT)
(72) Inventor: DE SÁ MARTINS, César André, P-4905-016 Aldreu (PT); MARTINS INÁCIO, Afonso Henrique, P-4750-352 Barcelos (PT)
(74) Representative: Patentree
(86) International application number: PCT/IB2015/055834
(87) International publication number: WO 2016/016867

(56) References cited:
- WO-A1-2012/136607
- WO-A1-2014/072015
- WO-A2-2009/086193
- US-A1- 2006 141 015

## Description

### Technical domain

The present disclosure relates to a process for obtaining and formulating functional silica particles and other materials with or without active ingredients for use in polymers, paints, mortars, ceramic, cement, textile, pharmaceutics, varnishes, paper, clays, cosmetics, sensors and effluents.

The active ingredients/functional compounds to be transported can be chosen from the following group: bactericides, fungicides, antimycotics, repellants, insecticides, pesticides, and larvicides.

### Background

WO2010114632A2 discloses a process for metal nanoparticle functionalization that allows them to have reversible aggregation/disaggregation properties. The composition is a nanoparticle functionalized with a coating material and the outer portion of the coating material is functionalized with polyethylene glycol (PEG).

US 2010/0136124 A1 discloses a process for drug release in the skin and describes a formulation within a particle-coated capsule meant to be applied to the skin, for example for the treatment of a disease. The described formulation comprises oil-based or aqueous droplets containing an active ingredient inside a nanoparticle coating, particularly silica nanoparticles.

WO2006102377A2 discloses a contrast process for use in Magnetic Resonance Imaging. It refers to magnetic nanoparticle functionalization with a functional group to be applied in the biomedical field, for example, in magnetic resonance imaging. The composition comprises: a functionalized magnetic nanoparticle, having a functional group with differential affinity with a tissue of the brain, and a pharmaceutically acceptable carrier.

WO2014/072015A1 discloses composite oxide-based materials for the release of biologically active agents, the size of the particles can range from 10 to 100 nm, especially 50 to 100 nm, or from 100 nm to 100 um; the particles are preferably silicon-oxide materials, with a mean diameter of 0.5 µm (i.e. 500 nm) to 500 um.

WO2012/136607A1 discloses a skin care formulation comprising: a dispersion or emulsion comprising at least one particle-polymer hybrid wherein the polymer-particle hybrid comprises at least one particle grafted with at least one polymer; and a cosmetically acceptable additive; the particle can contain a hydroxyl group and can be selected from SiO₂, TiO₂, ZnO, and has an average particle size in the range of from 10 nm to 1 micron, preferably from 100 nm to 1 micron.

WO2009086193A2 discloses a particle to be attached to a substrate, the particle comprising a silane binder and an active compound.

These facts are described to illustrate the technical problem solved by the embodiments of the present document.

### General Description

The present invention describes a functional particle for binding to a substrate comprising:
0.1 50 % (w/v) of a granule comprising oxide or hydroxide of an element selected from the following list: silica, magnesium, zinc, iron, copper, silver, aluminum, gold, titanium, or mixtures thereof having a size between 120nm-250nm;
5-50 % (w/v) of a binder comprising silane-based compounds which binds the outer granule to the substrate;
40-80 % (w/v) of a functional compound/active compound bound to the surface of the granule, to the binder, or to both;
wherein the functional compound is at least one of the following compounds: anti-mosquito/repellent/anti-insect, fungicide, antimicrobial/bactericide, antimycotic, larvicides, mechanical properties enhancement, magnetic properties, or mixtures thereof, among others; wherein the particle size range is between 120nm-250nm.

The particles described are functionalized with several active ingredients, which are on the surface, and binding agents, but which do not involve a capsule.

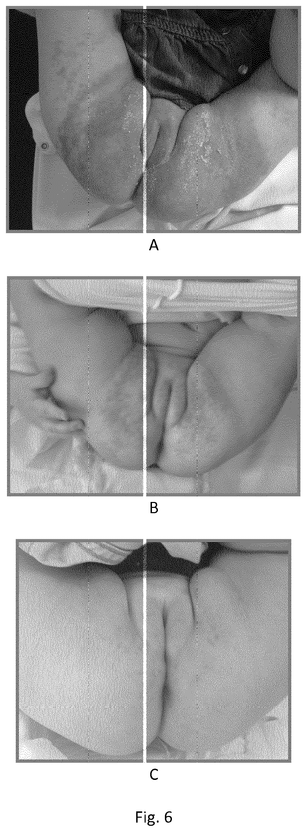

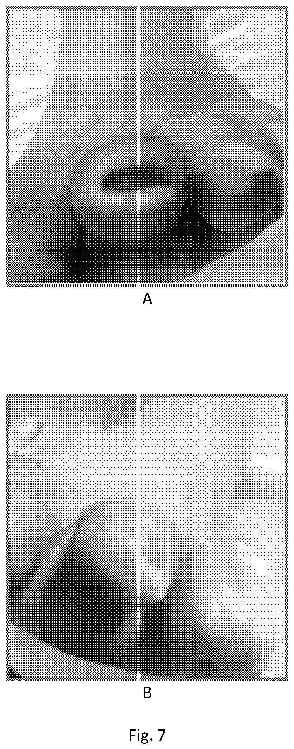

The functional particles increase the effect of the active compound (or functional compound) - see Figures 6/7 and also manage for the effect to prevail even after several uses and or washes - see Tables I-VIII.

The granule does not have a fixed shape and can be spherical, oval, irregular, porous. The size of the granule can be obtained by various methods, such as sol-gel and simple salts precipitation.

The size of the granules and/or particles can be determined by several conventional methods including electron microscopy - scanning electron microscope (SEM).

The particles have a particle size ranging from 120 nm-250 nm.

The substrate can be selected from the following list: polymers, paints, mortars, clays, cements, textiles, varnishes, paper, cosmetics, detergents, laminates, non-woven fabric, leathers, sensors and effluents.

The functional compound/active compound is at least one of the following: anti-mosquito/repellent/antiinsect, fungicide, antimicrobial/bactericide, antimycotic, dies, aromas, essential oils, larvicides, magnetics, or mixtures thereof.

The repellant can be selected from: ethyl butylacetylaminopropionate, permethrin, deltamethrin, p-3,8metano-diol, icaridin/saltidin, N,N-dimethyl-meta-toluamide (DEET), citronella oil, methylneodecanamide, fipronil, empenthrin, d-allethrin, piperonyl butoxide, transfluthrin, prallethrin, biphenothrin, pyriproxyfen, imidacloprid, brodifacoum, tetramethrin, bmidaclopride, bromadiolone, bumatetralyl, blocumafene, bipermethrin, citriodiol or mixtures thereof.

The fungicide can be selected from the following list: celery oil, chlorhexidine gluconate, benzalkonium chloride, zinc oxide, tea tree oil, octylisothiazolinone, di-n-decyldimethylammoniumchloride, phenoxyethanol, benzimidazole or mixtures thereof.

The antimicrobial can be selected among: benzoyl peroxide, celery oil, chlorhexidine gluconate, benzalkonium chloride, zinc oxide, tea tree oil, ortho-phenylphenol 2-benzyl-4-chlorophenol, cetyltrimethylammonium bromide, silver nitrate, zinc nitrate, copper nitrate, octylisothiazolinone, di-n-decyldimethylammoniumchloride, phenoxyethanol, chitosan or mixtures thereof.

The antimycotic can be selected from a list comprised by: ketoconazole, terbinafine, sertaconazole, clotrinazole, fluconazole, itraconazole, fluconazole, nystatin, celery oil, chlorhexidine gluconate, benzalkonium chloride, octylisothiazolinone, di-n-decyldimethylammoniumchloride or mixtures thereof.

The larvicide can be selected from a list comprised by: Methyl 4-hydroxybenzoate, temephos, diflubenzuron, novaluron, pyriproxyfen, diflubenzuron, methoprene or mixtures thereof.

In an embodiment the particles described in the present disclosure can further comprise vitamins, moisturizers, cosmetics, or mixtures thereof.

In an embodiment of the particles, the binder is selected from a list comprised by (3-glycidyloxypropyl)trimethoxysilane, octyltriethoxysilane, octyltrimethoxysilane, propyltriethoxysilane, propyltrimethoxysilane, 3-aminopropyltriethoxysilane, 3-aminopropyltrimethoxysilane, HYDROSIL 2909, HYDROSIL 2627, 3-glycidyloxypropyltriethoxysilane, Dynasylan F 8815, 3-methacryloxypropyltrimethoxysilane, Dynasylan^{®} HYDROSIL 2926, 3-aminopropyl-methyl-diethoxysilane, 3-[diethoxy(methyl)silyl]propan-1-amine, 3-glycidyloxypropylmethyldimethoxysilane, 3-glycidyloxypropylmethyldiethoxysilane or mixtures thereof.

Another aspect of the present invention describes a composition comprising the particles described in the present disclosure.

In an embodiment the composition includes:
- 20-50% (w/v) of a binder;
- 50-80% (w/v) of the active compound/functional compound;
- 0.1-20% (w/v) of a granule comprising oxide or hydroxide selected from the following list: silica, magnesium, zinc, iron, copper, silver, aluminum, gold, titanium or mixtures thereof having a size between 120nm-250nm.

In an embodiment the composition can further comprise a softener, dispersant, solvent, or mixtures thereof.

The composition can be used in medicine, particularly in the treatment of skin infections, dermatitis, wounds, ulcers including diabetic foot (in diapers, sanitary towels, socks or swabs).

Another aspect of the present invention concerns the use of the functional particles described and/or composition to functionalize substrates and improve the effect of the active compound.

Another aspect of the present invention concerns articles comprising the particles and/or compositions described. In particular, the articles are: clothing, or footwear, or headwear, or mortar, or ceramic articles, or clay or rubber articles, or polymers, or paints, or cement, or varnishes, or paper, or health, or sensors, or chemicals, or cosmetics or pharmaceuticals.

Also described is a method for obtaining granules comprising the following steps:
- dissolving in water a salt, a nitrate, or sulphate, or chloride of an element selected from the following list: silica, magnesium, zinc, iron, copper, silver, aluminum, gold, titanium or mixtures thereof under stirring;
- optionally adding 01-10g of a surfactant to the previous solution;
- adding an alkaline solution to precipitate the previous solution under stirring for 30 min-1.5 h and obtaining the granules;
- adding to the granules previously obtained the selected binders and active ingredients.

An embodiment of the method described comprises dissolving in water 40 ml water, 6-30g sodium metasilicate under stirring.

In an embodiment of the method described salts can be:
- a pentahydrate sodium metasilicate; or
- a nitrate of the elements selected from the following list: silica, magnesium, calcium, iron, copper, silver, zinc, aluminum, gold, titanium or mixtures thereof; or
- a sulfate of the elements selected from the following list: silica, magnesium, calcium, iron, copper, silver, zinc, aluminum, titanium or mixtures thereof; or
- a chloride of elements selected from the following list: silica, silver, calcium, magnesium, iron, zinc, copper, aluminum, gold, titanium, and mixtures thereof or mixtures thereof.

In an embodiment of the method described the surfactant is selected from the following list: polyether, polyethylene glycol (PEG), polyethylene oxide (PEO), polyethylene oxide-co-polypropylene oxide (PPO), co-polyethylene oxide, polyvinyl alcohol (PVA), poly(vinyl pyrrolidinone) (PVP), polyethylene oxide-polypropylene oxide-polyethylene-oxide (PEO-PPO-PEO), cetrimonium bromide, sodium dodecyl sulfate (SDS), or mixtures thereof.

In an embodiment of the method described alkaline or acidic solutions are used in the precipitation.

The particles and/or compositions can be applied by spraying, dipping or additivation.

Throughout the specification and claims the word "comprising" and variations thereof, are not intended to exclude other technical features, additives, components, or steps.

### Description of the Drawings

For an easier understanding of the solution, drawings are herein attached, which represent preferred embodiments and which, however, are not intended to limit the scope of the present application.

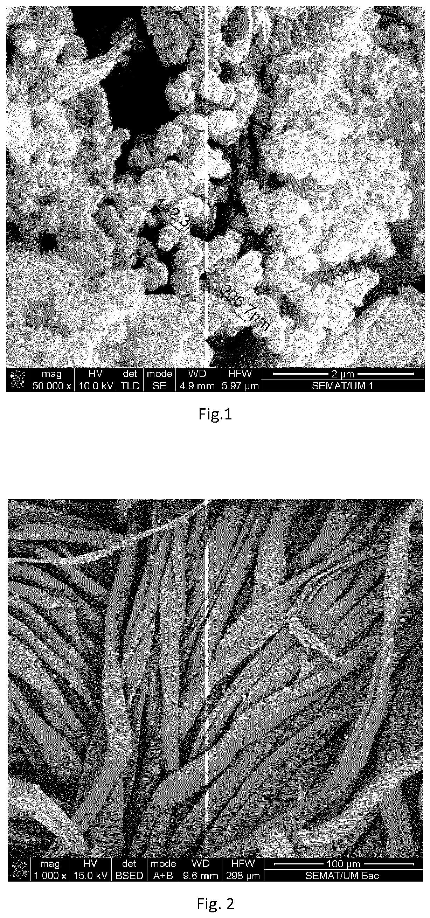

**Figure 1****:** Representation of mosquito repellent silica particles in ceramic substrate.
**Figure 2****:** Representation of bactericide silica particles in textile substrate.

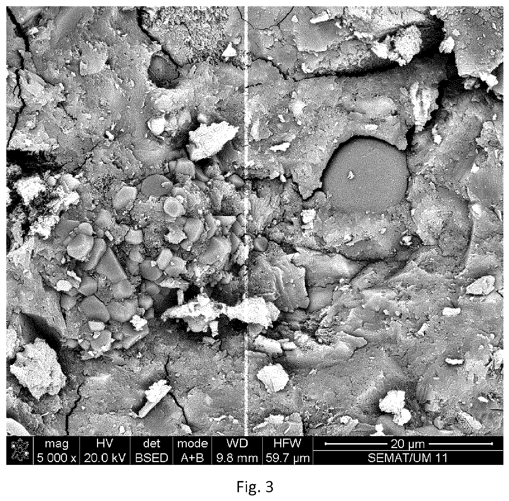

**Figure 3****:** Representation of aluminum particles in cement substrate.

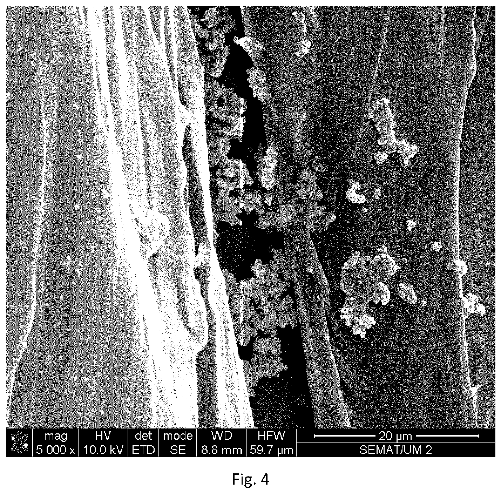

**Figure 4****:** Representation of mite repellent silica particles in textile substrate.

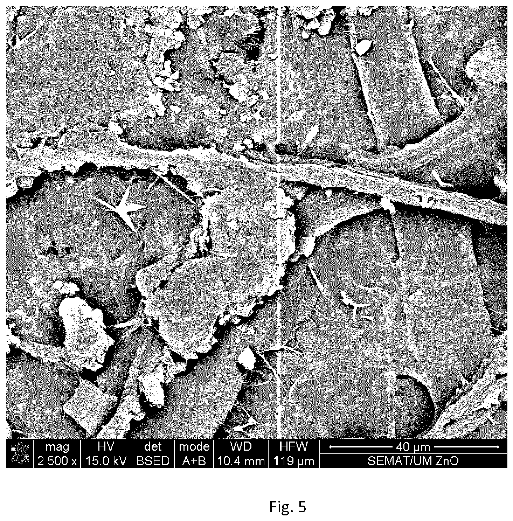

**Figure 5****:** Representation of zinc oxide particles in paper.
**Figure 6****:** Representation of dermatitis treatment with the application of the particles from example in Table III bactericidal in diapers; wherein Figure A represents the patient's skin after using conventional diapers and drugs, Figure B represents the patient's skin after using diapers with the particles described in Table III for 10 days; and Figure C represents the patient's skin after using the diapers with the particles described in Table III for 20.
**Figure 7****:** Representation of ulcer treatment in diabetic foot with the application of the particles from example in Table III in conventional socks; wherein Figure A represents the patient's foot with the use of conventional socks and drugs, Figure B is represents the patient's skin after using socks with particles described in Table III for 1 month.

### Detailed description

Also described is a process for obtaining functional particles with active ingredients and applications thereof in the textile field (as bactericidal, fungicidal, repellent, anti-mosquito, antimycotic, agricultural network (anti-insect). The present particles can also be used in mortars, cements, paints, varnishes, floors, pools (as a fungicide, repellant, larvicide, hydrophobic, for tensile strength and better frame connection), polymers (such as bactericidal, biodegradability, UV protection, gas impermeability, gas permeability, electrical conductivity, anti-fire, hydrophobic). Additionally they can be used for wastewater treatment, in pharmaceutical industry for drug delivery, paper (as bactericidal, fungicidal, anti-odor, repellent, antimycotic, anti-fire, hydrophobic, magnetic) and electronics.

The particles can be used to functionalize the most varied materials in a simple application method with great efficiency and durability.

In an embodiment, the process for obtaining the 120nm-250nm particles comprises the following steps:
a) Dissolving in 40 mL water 2g-60g, more preferably 6-30g pentahydrate sodium metasilicate or nitrate of the elements selected from the following list:
   calcium, iron, copper, silver, zinc, aluminum, gold, titanium, silica and mixtures thereof;
   or sulfate of elements selected from the following list:
      magnesium, calcium, iron, copper, silver, zinc, aluminum, titanium, silica and mixtures thereof;
      or chloride of elements selected from the following list:
         silver, calcium, magnesium, iron, zinc, copper, aluminum, gold, titanium, silica and mixtures thereof under stirring;
b) Optionally adding, under stirring for 1-30 minutes, 0.1g - 10g surfactant from the following list: Triton^{™} X100, Tween 20, 40, 60 and 80, polyether, polyethylene glycol (PEG), polyethylene oxide (PEO), polyethylene oxide-co-polypropylene oxide (PPO), polyvinyl alcohol (PVA), poly(vinyl pyrrolidinone) (PVP), bromide cetrimonium, sodium dodecyl sulfate (SDS), or mixtures thereof to the mixture resulting from step (a).
c) Adding 1-100 mL of active ingredient/functional compound or mixture of active ingredients/functional compounds, preferably able to be diluted with water, alcohols or solvents; even more preferably, in order to improve the bonding among the particles and active ingredient, 1-200mL silane is added selected from the following group: (3-Glycidyloxypropyl)trimethoxysilane, octyltriethoxysilane, octyltrimethoxysilane, propyltriethoxysilane, propyltrimethoxysilane, 3-aminopropyltriethoxysilane, 3-aminopropyltrimethoxysilane, HYDROSIL 2909, HYDROSIL 2627, 3-glycidyloxypropyltriethoxysilane, Dynasylan F 8815, 3-methacryloxypropyltrimethoxysilane, Dynasylan^{®} HYDROSIL 2926, 3-aminopropyl-methyl-diethoxysilane, 3-[diethoxy(methyl)silyl]propan-1-amine, 3-glycidyloxypropylmethyldimethoxysilane, 3-glycidyloxypropylmethyldiethoxysilane or mixtures thereof.
d) Adding an alkaline solution (eg, sodium phosphate, ammonia, sodium hydroxide among other salts) for precipitating the above solution under stirring for 30 minutes to 1.5 hours but preferably 50-80 minutes, preferably a solution with 20 mL water with 1g to 100 g ammonium chloride.

In an embodiment, the obtention process can be performed only with steps a) and d) obtaining particles for mixing with binders and active ingredients.

In some cases, depending on the application, the particles can be dried or filtered.

The active ingredient can be selected from the following group:
- repellents such as ethyl butylacetylaminopropionate, permethrin, deltamethrin, p-3,8metano-diol, icaridin/saltidin, N,N-dimethyl-meta-toluamide (DEET), citronella oil, methylneodecanamide, fipronil, empenthrin, d-allethrin, piperonyl butoxide, transfluthrin, prallethrin, biphenothrin, pyriproxyfen, imidacloprid, brodifacoum, tetramethrin, bmidaclopride, bromadiolone, bumatetralyl, blocumafene, bipermethrin, citriodiol or mixtures thereof;
- fungicides such as celery oil, chlorhexidine gluconate, benzalkonium chloride, zinc oxide, tea tree oil, octylisothiazolinone, di-n-decyldimethylammoniumchloride, phenoxyethanol, benzimidazole or mixtures thereof;
- antimicrobial, such as benzoyl peroxide, celery oil, chlorhexidine gluconate, benzalkonium chloride, zinc oxide, tea tree oil, ortho phenyl phenol, 2-benzyl-4-chlorophenol, cetyltrimethylammonium bromide, silver nitrate, zinc nitrate, copper nitrate, octylisothiazolinone, di-n-decyldimethylammoniumchloride, phenoxyethanol, chitosan or mixtures thereof;
- antimycotics such as ketoconazole, terbinafine, sertaconazole, clotrinazole, fluconazole, itraconazole, fluconazole, nystatin, celery oil, chlorhexidine gluconate, benzalkonium chloride, octylisothiazolinone, di-n-decyldimethylammoniumchloride or mixtures thereof;
- larvicides such as Methyl 4-hydroxybenzoate, temephos, diflubenzuron, novaluron, pyriproxyfen, diflubenzuron, methoprene or mixtures thereof;
- anti-insects such as methylneodecanamide, fipronil, empenthrin, d-allethrin, piperonyl butoxide, transfluthrin, prallethrin, cyphenothrin, pyriproxyfen, imidacloprid, brodifacoum, tetramethrin, imidacloprid, bromadiolone, coumatetralyl, flocoumafen, or mixtures thereof.

These active ingredients can be mixed in active ingredient/binder group ratios (w/v) of 10/90 or 20/80 or 30/70 or 40/60 or 50/50 or 60/40 or 70/30 or 80/20 or 90/10, and this concentrate can also be diluted in solvents, for example, in water or alcohols w/v at 10/90 or 20/80 or 30/70 or 40/60 or 50/50 or 60/40 or 70/30 or 80/20 or 90/10.

The functionalized particles and/or compositions described can be sprayed onto the surfaces of various materials.

The functionalized particles and/or compositions described can this solution be further mixed with paints, mortars, cements, textiles, pharmaceuticals, varnishes, electronics, polymers, sensors, civil construction, laminates, paper, paper pulp, diapers, swabs, sanitary towels, detergents, among others.

Effect of application of the particles described in the present disclosure for several applications, add 1% to 50% w/v.

**Table I - Shows tests conducted with Aedis Egypti mosquitos using the particles herein described, namely with silica/octyltriethoxysilane/ethyl butylacetylaminopropionate:**

| Sample | % Repellency |
|---|---|
| Textile without particles | 0% |
| Textile with particles described, 0 washings | 100% |
| Textile with particles described, 50 washings | 91% |
| Textile with particles described, 80 washings | 84% |
| Textile with particles described, 100 washings | 75% |

**Table II - Shows tests conducted with Aedis Egypti mosquitos using the particles herein described, namely with silica/3-aminopropyltriethoxysilane/ethyl butylacetylaminopropionate:**

| Sample | % Repellency |
|---|---|
| Paint with no particles described | 0% |
| Paint with particles described | 99.99% |

**Table III - Shows tests performed with Staphylococcus aureus in diapers and socks using the particles herein described, particularly with silica/ organofunctional siloxane oligomer reactive in water/benzalkonium chloride (see effect in figures 6/7); in these cases, particles were applied by spraying (spray) in conventional diapers and socks:**

| Sample | % Effectiveness |
|---|---|
| Textile without particles | 0% |
| Textile with particles described, 20 washings | 91% |
| Textile with particles described, 50 washings | 70% |

**Table IV - Tests conducted with Staphylococcus aureus using the particles herein described, in particular silica/ organofunctional siloxane oligomer reactive in water/chlorhexidine gluconate/benzalkonium chloride:**

| Sample | % Effectiveness |
|---|---|
| Paint with no particles described | 0% |
| Paint with particles described | 99.99% |

**Table V - Tests conducted with Aspergillus niger using the particles herein described in conventional diapers and conventional socks, in particular silica/ organofunctional siloxane oligomer reactive in water/chlorhexidine gluconate/ benzalkonium chloride (see effect in figures 6/7):**

| Sample | % Effectiveness |
|---|---|
| Paint with no particles described | 0% |
| Paint with particles described | 99.99% |

**Table VI - Tests conducted with Dermatophagoides pteronyssinus mite using the particles herein described in particular silica/octyltriethoxysilane/Ethyl Butylacetylaminopropionate:**

| Sample | % Repellency |
|---|---|
| Textile without particles | 0% |
| Textile with particles described | 92.25% |

**Table VII - Tests conducted with Dermatophagoides farinae mite using the particles herein described in particular silica/octyltriethoxysilane/Ethyl Butylacetylaminopropionate:**

| Sample | % Repellency |
|---|---|
| Textile without particles | 0% |
| Textile with particles described | 85.8% |

**Table VIII - Tests conducted with Staphylococcus aureus using the particles herein described, in particular silica/ organofunctional siloxane oligomer reactive in water/benzalkonium chloride:**

| Sample | % Effectiveness |
|---|---|
| Paper with no particles described | 0% |
| Paper with particles described | 99.99% |

Despite several washes which the material is subjected to, it has been found that functionality is maintained after such several washes, these properties being due to the structure of the particles, obtetion process, composition and obtention method thereof.

### Synthesis Examples

### Example 1: Silica Particles (not according to the invention)

1g to 20g pentahydrate sodium metasilicate is added to 40 mL water and dissolved so as to obtain an emulsion.

For the precipitation of the emulation, a 20 mL water side solution is added and 1g to 50g ammonium chloride, then the above solution is added and left under stirring for 5 minutes to 120 minutes.

### Example 2: Synthesis of zinc hydroxide particles (not according to the invention)

In 40mL water 1 g to 200 g zinc nitrate or zinc sulfate is dissolved and stirred to complete dissolution.

Then, 0.1g to 5g Triton X100 is added and stirred for 1 to 30 minutes.

For the precipitation of the emulation, a 20 mL water side solution is prepared and 1g to 50g ammonium chloride or 5 mL to 100 mL 50% NaOH is added, then the previous solution is added and left under stirring for 5 minutes to 120 minutes.

### Example 3: Synthesis of silica particles with other materials (not according to the invention).

1g to 20g pentahydrate sodium metasilicate is added to 40 mL water and dissolved.

Then the above solution is added with a 10mL to 100mL water solution with 1g to 100g nitrate of elements selected from the following list:
magnesium, calcium, iron, copper, silver, zinc, aluminum, gold, titanium, and mixtures thereof;
or sulfate of elements selected from the following list:
   magnesium, calcium, iron, copper, silver, zinc, aluminum, titanium, and mixtures thereof;
   or chloride of elements selected from the following list: silver, calcium, magnesium, iron, zinc, copper, aluminum, gold, titanium, and mixtures thereof.

For the precipitation of the emulation, a 20 mL water side solution is prepared and 1g to 50g ammonium chloride or 5 mL to 100 mL 50% NaOH is added, then the previous solution is added and left under stirring for 5 minutes to 120 minutes.

### Example 4: Synthesis of antimycotic silica particles

1g to 20g pentahydrate sodium metasilicate is added to 40 mL water and dissolved.

Then the above solution 1 to 100g ketoconazole and 1 to 10mL (3-Glycidyloxypropyl)trimethoxysilane is added.

For the precipitation of the emulation, a 20 mL water side solution is prepared and 1g to 50g ammonium chloride is added and the previous solution is added and left under stirring for 5 minutes to 120 minutes.

### Example 5: Active ingredient (not according to the invention)

(3-Glycidyloxypropyl)trimethoxysilane, octyltriethoxysilane, octyltrimeth-oxysilane, propyltriethoxysilane, propyltrimethoxysilane, 3-aminopropyltriethoxysilane, 3-aminopropyltrimethoxysilane, HYDROSIL 2909, HYDROSIL 2627, (3-glycidyloxy)propyltriethoxysilane, Dynasylan F 8815, 3-methacryloxypropyltrimethoxysilane, Dynasylan^{®} HYDROSIL 2926, 3-aminopropyl-methyl-diethoxysilane, 3-[diethoxy(methyl)silyl]propan-1-amine, 3-Glycidyloxypropylmethyldimethoxysilane, 3-Glycidyloxypropylmethyldiethoxysilane or mixtures thereof with Ethyl Butylacetylaminopropionate, among other active ingredients are mixed in a w/v ratio ranging 10/90 or 20/80 or 30/70 or 40/60 or 50/50 or 60/40 or 70/30 or 80/20 or 90/10, the concentrate can also be diluted in water, alcohols or solvents at a w/v ratio ranging 10/90 or 20/80 or 30/70 or 40/60 or 50/50 or 60/40 or 70/30 or 80/20 or 90/10.
All these Examples can be applied:
The application of these particles can be applied by spraying or dipping or additivation.

Dispersing or dry mixture with polymers, paints, mortars, cements, paper, paper pulp, detergents, textile, varnishes, ceramics, cosmetics, electronics, sensors, glass, civil construction, laminates, diapers, sanitary towels, swabs, medical devices, pharmaceutical articles, among others.

### Example Application in textile stretching, tumbler or washing machine:

Amount in examples 1,2,3,4,5: from 1g/L to 200g/L
Binder: 1g/L to 300g/L (e.g. ((3-Glycidyloxypropyl)trimethoxysilane, octyltriethoxysilane, octyltrimethoxysilane, propyltriethoxysilane, propyltrimethoxysilane, 3-aminopropyltriethoxysilane, 3-aminopropyltrimethoxysilane, HYDROSIL 2909, HYDROSIL 2627, 3-glycidyloxypropyltriethoxysilane, Dynasylan F 8815, 3-methacryloxypropyltrimethoxysilane, Dynasylan^{®} HYDROSIL 2926, 3-aminopropyl-methyl-diethoxysilane, 3-[diethoxy(methyl)silyl]propan-1-amine, 3-glycidyloxypropylmethyldimethoxysilane, 3-glycidyloxypropylmethyldiethoxysilane or mixtures thereof).

Drying is carried out between 20 °C to 200 °C.

### Application Example in Paints and Varnishes:

Amount in examples 1,2,3,4,5: between 1 g/L to 300 g/L, mix and apply paint or varnish.

The embodiments described above are combinable.

## Claims

1. Functional particle for binding to a substrate comprising:
0.1 - 50 % (w/v) of a granule comprising oxide or hydroxide of an element selected from the following list: silica, magnesium, zinc, iron, copper, silver, aluminum, gold, titanium, or mixtures thereof having a size between 120 nm- 250 nm;
5-50 % (w/v) of a binder comprising silane-based compounds which binds the outer granule to the substrate;
40-80 % (w/v) of a functional compound/active compound bound to the surface of the granule, to the binder, or to both;
wherein the functional compound/active compound is at least one of the following compounds: anti-mosquito/repellent/anti-insects, fungicide, antimicrobial/bactericide, antimycotic, larvicides, , or mixtures thereof;
wherein the particle size range is between 120nm-250nm.

2. Particle according to the preceding claim comprising:
20%-50% (w/v) of a binder,
50-80% (w/v) of an active compound/functional compound,
0.1%-20% (w/v) of a granule comprising oxide or hydroxide of an element selected from the following list: silica, magnesium, zinc, iron, copper, silver, aluminum, gold, titanium or mixtures thereof having a size between 120 nm - 250 nm.

3. Particle according to the preceding claims wherein the binder is selected from a list comprised by (3-glycidyloxypropyl) trimethoxysilane, octyltriethoxysilane, octyltrimeth-oxysilane, propyltriethoxysilane, propyltrimethoxysilane, 3-aminopropyltriethoxysilane, 3-aminopropyltrimethoxysilane, organofunctional oligosiloxane reactive in water, fluoroalilfunctional oligosiloxane, 3-glycidyloxypropyltriethoxysilane, 3-methacryloxypropyltrimethoxysilane, 3-aminopropyl-methyl-diethoxysilane, 3-[diethoxy(methyl)silyl]propan-1-amine, 3-glycidyloxypropylmethyldimethoxysilane, 3 - glycidyloxypropylmethyldiethoxysilane, or mixtures thereof.

4. Particle according to the preceding claims wherein the binder is (3-glycidyloxypropyl) trimethoxysilane, octyltriethoxysilane, 3-aminopropyltriethoxysilane, organofunctional oligosiloxane reactive in water, fluoroalilfunctional oligosiloxane, 3-aminopropyl-methyl-diethoxysilane, 3-[diethoxy(methyl)silyl]propan-1-amine, 3-glycidyloxypropylmethyldimethoxysilane, 3-glycidyloxypropylmethyldiethoxysilane, or mixtures thereof.

5. Particle according to the preceding claims wherein the **repellant** is selected from: ethyl butylacetylaminopropionate, permethrin, deltamethrin, p-3,8metano-diol, icaridin/saltidin, N,N-dimethyl-meta-toluamide, citronella oil, methylneodecanamide, fipronil, empenthrin, d-allethrin, piperonyl butoxide, transfluthrin, prallethrin, biphenothrin, pyriproxyfen, imidacloprid, brodifacoum, tetramethrin, bmidaclopride, bromadiolone, bumatetralyl, blocumafene, bipermethrin, citriodiol or mixtures thereof.

6. Particle according to the preceding claims wherein the **fungicide** is selected from the following list celery oil, chlorhexidine gluconate, benzalkonium chloride, zinc oxide, tea tree oil, octylisothiazolinone, di-n-decyldimethylammoniumchloride, phenoxyethanol, benzimidazole or mixtures thereof.

7. Particle according to the preceding claims wherein the **antimicrobial** is selected among: benzoyl peroxide, celery oil, chlorhexidine gluconate, benzalkonium chloride, zinc oxide, tea tree oil, ortho-phenylphenol 2-benzyl-4 -chlorophenol, cetyltrimethylammonium bromide, silver nitrate, zinc nitrate, copper nitrate, octylisothiazolinone, di-n-decyldimethylammoniumchloride, phenoxyethanol, chitosan or mixtures thereof.

8. Particle according to the preceding claims wherein the **antimycotic** is selected from a list comprised by: ketoconazole, terbinafine, sertaconazole, clotrinazol, fluconazole, itraconazole, fluconazole, nystatin, celery oil, chlorhexidine gluconate, benzalkonium chloride, octylisothiazolinone, di-n-decyldimethylammoniumchloride or mixtures thereof.

9. Particle according to the preceding claims wherein the larvicide is selected from a list comprised by: Methyl 4-hydroxybenzoate, temephos, diflubenzuron, novaluron, pyriproxyfen, diflubenzuron, methoprene or mixtures thereof.

10. Particle according to the preceding claims further comprising vitamins, moisturizers, cosmetics, essential oils or mixtures thereof.

11. Composition comprising the particles described in the preceding claims.

12. Composition according to the preceding claim for use as a medicament, in particular for the treatment of skin infections, dermatitis, wounds, ulcers, namely diabetic foot.

13. Composition according to the preceding claim further comprising a softener, a dispersant, solvent or mixtures thereof.

14. Non-therapeutic use of particles according the description in claims 1-10 or of the composition as described in claims 11-13 as a functionalizer of various materials and enhancer of the effect of the active compound over time.

15. Articles comprising the particles and/or compositions described in the preceding claims; in particular wherein the articles are: clothing, footwear, headwear, mortars, polymers, ceramic clay, rubber, paints, cements, pharmaceuticals, varnishes, paper, paper pulp, medical devices, cosmetics, sensors, detergents, laminates, diapers, or sanitary towels.

## Patentansprüche

1. Funktioneller Partikel zur Bindung an ein Substrat, umfassend:
0,1-50 % (w/v) eines Granulats, umfassend Oxid oder Hydroxid eines Elements, ausgewählt aus der folgenden Liste: Siliciumdioxid, Magnesium, Zink, Eisen, Kupfer, Silber, Aluminium, Gold, Titan oder Mischungen davon mit einer Größe zwischen 120 nm-250 nm;
5-50 % (w/v) eines Bindemittels, umfassend Verbindungen auf Silanbasis, das das äußere Granulat an das Substrat bindet;
40-80 % (w/v) einer funktionellen Verbindung/eines Wirkstoffs, die/der an die Oberfläche des Granulats, an das Bindemittel oder an beide gebunden ist;
wobei die funktionelle Verbindung/der Wirkstoff mindestens eine der folgenden Verbindungen ist: mückenabweisend/repellent/insektenabweisend, fungizid, antimikrobiell/bakterizid, antimykotisch, larvizid oder Mischungen davon;
wobei die Partikelgröße im Bereich zwischen 120 nm-250 nm liegt.

2. Partikel nach dem vorangehenden Anspruch, umfassend:
20-50 % (w/v) eines Bindemittels,
50-80 % (w/v) eines Wirkstoffs/einer funktionellen Verbindung,
0,1-20 % (w/v) eines Granulats, umfassend Oxid oder Hydroxid eines Elements, ausgewählt aus der folgenden Liste: Siliciumdioxid, Magnesium, Zink, Eisen, Kupfer, Silber, Aluminium, Gold, Titan oder Mischungen davon mit einer Größe zwischen 120 nm-250 nm.

3. Partikel nach den vorangehenden Ansprüchen, wobei das Bindemittel ausgewählt ist aus einer Liste, umfassend (3-Glycidyloxypropyl)trimethoxysilan, Octyltriethoxysilan, Octyltrimethoxysilan, Propyltriethoxysilan, Propyltrimethoxysilan, 3-Aminopropyltriethoxysilan, 3-Aminopropyltrimethoxysilan, organofunktionelles, in Wasser reaktives Oligosiloxan, fluoraliphatisches Oligosiloxan, 3-Glycidyloxypropyltriethoxysilan, 3-Methacryloxypropyltrimethoxysilan, 3-Aminopropyl-methyl-diethoxysilan, 3-[Diethoxy(methyl)silyl]propan-1-amin, 3-Glycidyloxypropylmethyldimethoxysilan, 3-Glycidyloxypropylmethyldiethoxysilan oder Mischungen davon.

4. Partikel nach den vorangehenden Ansprüchen, wobei das Bindemittel (3-Glycidyloxypropyl)trimethoxysilan, Octyltriethoxysilan, 3-Aminopropyltriethoxysilan, organofunktionelles, in Wasser reaktives Oligosiloxan, fluoraliphatisches Oligosiloxan, 3-Aminopropyl-methyl-diethoxysilan, 3-[Diethoxy(methyl)silyl]propan-1-amin, 3-Glycidyloxypropylmethyldimethoxysilan, 3-Glycidyloxypropylmethyldiethoxysilan oder Mischungen davon.

5. Partikel nach den vorangehenden Ansprüchen, wobei das Repellent ausgewählt ist aus: Ethyl-butylacetylaminopropionat, Permethrin, Deltamethrin, p-Menthane-3,8-diol, Icaridin/Saltidin, N,N-Dimethyl-meta-Toluamid, Citronellaöl, Methylneodecanamid, Fipronil, Empenthrin, d-Allethrin, Piperonylbutoxid, Transfluthrin, Prallethrin, Biphenothrin, Pyriproxyfen, Imidacloprid, Brodifacoum, Tetramethrin, Bmidacloprid, Bromadiolon, Bumatetralyl, Blocumafene, Bipermethrin, Citriodiol oder Mischungen davon.

6. Partikel nach den vorangehenden Ansprüchen, wobei das Fungizid aus der folgenden Liste ausgewählt ist: Sellerieöl, Chlorhexidingluconat, Benzalkoniumchlorid, Zinkoxid, Teebaumöl, Octylisothiazolinon, Di-n-decyldimethylammoniumchlorid, Phenoxyethanol, Benzimidazol oder Mischungen davon.

7. Partikel nach den vorangehenden Ansprüchen, wobei das antimikrobielle Mittel ausgewählt ist aus: Benzoylperoxid, Sellerieöl, Chlorhexidingluconat, Benzalkoniumchlorid, Zinkoxid, Teebaumöl, ortho-Phenylphenol, 2-Benzyl-4-chlorphenol, Cetyltrimethylammoniumbromid, Silbernitrat, Zinknitrat, Kupfernitrat, Octylisothiazolinon, Di-n-decyldimethylammoniumchlorid, Phenoxyethanol, Chitosan oder Mischungen davon.

8. Partikel nach den vorangehenden Ansprüchen, wobei das Antimykotikum ausgewählt ist aus einer Liste, umfassend: Ketoconazol, Terbinafin, Sertaconazol, Clotrinazol, Fluconazol, Itraconazol, Fluconazol, Nystatin, Sellerieöl, Chlorhexidin-Gluconat, Benzalkoniumchlorid, Octylisothiazolinon, Di-n-decyldimethylammoniumchlorid oder Mischungen davon.

9. Partikel nach den vorangehenden Ansprüchen, wobei das Larvizid aus einer Liste ausgewählt ist, umfassend: Methyl-4-hydroxybenzoat, Temephos, Diflubenzuron, Novaluron, Pyriproxyfen, Diflubenzuron, Methopren oder Mischungen davon.

10. Partikel nach den vorangehenden Ansprüchen, ferner umfassend Vitamine, Feuchtigskeitspender, Kosmetika, ätherische Öle oder Mischungen davon.

11. Zusammensetzung, umfassend die in den vorangehenden Ansprüchen beschriebenen Partikel.

12. Zusammensetzung nach dem vorangehenden Anspruch zur Verwendung als Arzneimittel, insbesondere zur Behandlung von Hautinfektionen, Dermatitis, Wunden, Geschwüren, insbesondere eines diabetischen Fußsyndroms.

13. Zusammensetzung nach dem vorangehenden Anspruch, ferner umfassend einen Weichmacher, ein Dispergiermittel, ein Lösungsmittel oder Mischungen davon.

14. Nicht-therapeutische Verwendung von Partikeln nach der Beschreibung in den Ansprüchen 1-10 oder der Zusammensetzung nach der Beschreibung in den Ansprüchen 11-13 als Funktionalisierungsmittel verschiedener Materialien und Verstärker der Wirkung des Wirkstoffs im Laufe der Zeit.

15. Gegenstände, umfassend die in den vorangehenden Ansprüchen beschriebenen Partikel und/oder Zusammensetzungen, wobei die Gegenständen insbesondere sind: Kleidung, Schuhwerk, Kopfbedeckungen, Mörtel, Polymere, keramischer Ton, Gummi, Farben, Zemente, Arzneimittel, Lacke, Papier, Papierzellstoff, medizinische Geräte, Kosmetika, Sensoren, Reinigungsmittel, Laminate, Windeln oder Damenbinden.

## Revendications

1. Particule fonctionnelle destinée à être liée à un substrat comprenant :
0,1-50% (poids/volume) d'un granule comprenant un oxyde ou hydroxyde d'un élément choisi parmi la liste suivante : silice, magnésium, zinc, fer, cuivre, argent, aluminium, or, titane, ou des mélanges de ceux-ci ayant une taille située entre 120nm et 250nm ;
5-50% (poids/volume) d'un liant comprenant des composés à base de silane qui lie le l'extérieur du granule au substrat ;
40-80% (poids/volume) d'un composé fonctionnel/composé actif lié à la surface du granule, au liant, ou aux deux ;
dans laquelle le composé fonctionnel/composé actif est au moins un des composés suivants : anti-moustiques/répulsif/anti-insectes, fongicide, antimicrobien/bactéricide, antimycosique, larvicides, ou des mélanges de ceux-ci ;
dans laquelle la taille de particule varie entre 120nm et 250nm.

2. Particule selon la revendication précédente comprenant :
20-50% (poids/volume) d'un liant ;
50-80% (poids/volume) d'un composé actif/composé fonctionnel ;
0,1%-20% (poids/volume) d'un granule comprenant un oxyde ou hydroxyde d'un élément choisi parmi la liste suivante : silice, magnésium, zinc, fer, cuivre, argent, aluminium, or, titane, ou des mélanges de ceux-ci ayant une taille située entre 120nm et 250nm.

3. Particule selon les revendications précédentes dans laquelle le liant est choisi parmi une liste comprenant un (3-glycidyloxypropyl)triméthoxysilane, octyltriéthoxysilane, octyltriméthoxysilane, propyltriéthoxysilane, propyltriméthoxysilane, 3-aminopropyltriéthoxysilane, 3-aminopropyltriméthoxysilane, oligosiloxane organofonctionnel réactif dans l'eau, oligosiloxane fluoroalilfonctionnel, 3-glycidyloxypropyltriéthoxysilane, 3-méthacryloxypropyltriméthoxysilane, 3-aminopropyl-méthyl-diéthoxysilane, 3-[diethoxy(méthyl)silyl]propan-1-amine, 3-glycidyloxypropylméthyldiméthoxysilane, 3-glycidyloxypropylméthyldiéthoxysilane, ou des mélanges de ceux-ci.

4. Particule selon les revendications précédentes dans laquelle le liant est un (3-glycidyloxypropyl)triméthoxysilane, octyltriéthoxysilane, 3-aminopropyltriéthoxysilane, oligosiloxane organofonctionnel réactif dans l'eau, oligosiloxane fluoroalilfonctionnel, 3-aminopropyl-méthyl-diéthoxysilane, 3-[diethoxy(méthyl)silyl]propan-1-amine, 3-glycidyloxypropylméthyldiméthoxysilane, 3-glycidyloxypropylméthyldiéthoxysilane, ou des mélanges de ceux-ci.

5. Particule selon les revendications précédentes dans laquelle le répulsif est choisi parmi : le butylacétylaminopropionate d'éthyle, la perméthrine, la deltaméthrine, le p-menthane-3,8-diol, l'icaridine/saltidine, le N,N-diméthyl-méta-toluamide, l'huile de citronnelle, le méthylnéodécanamide, le fipronil, l'empenthrine, la d-alléthrine, le butoxyde de pipéronyle, la transfluthrine, la pralléthrine, la biphénothrine, le pyriproxyfène, l'imidaclopride, le brodifacoum, la tétraméthrine, le bmidaclopride, la bromadiolone, le bumatétralyl, le blocumafène, la biperméthrine, le citriodiol ou des mélanges de ceux-ci.

6. Particule selon les revendications précédentes dans laquelle le fongicide est choisi parmi la liste suivante : huile de céleri, gluconate de chlorhexidine, chlorure de benzalkonium, oxyde de zinc, huile d'arbre à thé, octylisothiazolinone, chlorure de di-n-décyldiméthylammonium, phénoxyéthanol, benzimidazole ou des mélanges de ceux-ci.

7. Particule selon les revendications précédentes dans laquelle l'antimicrobien est choisi parmi : le peroxyde de benzoyle, l'huile de céleri, le gluconate de chlorhexidine, le chlorure de benzalkonium, l'oxyde de zinc, l'huile d'arbre à thé, l'orthophénylphénol, le 2-benzyl-4-chlorophénol, le bromure de cétyltriméthylammonium, le nitrate d'argent, le nitrate de zinc, le nitrate de cuivre, l'octylisothiazolinone, le chlorure de di-n-décyldiméthylammonium, le phénoxyéthanol, le chitosane ou des mélanges de ceux-ci.

8. Particule selon les revendications précédentes dans laquelle l'antimycosique est choisi parmi une liste comprenant : le kétoconazole, la terbinafine, le sertaconazole, le clotrinazole, le fluconazole, l'itraconazole, le fluconazole, la nystatine, l'huile de céleri, le gluconate de chlorhexidine, le chlorure de benzalkonium, l'octylisothiazolinone, le chlorure de di-n-décyldiméthylammonium ou des mélanges de ceux-ci.

9. Particule selon les revendications précédentes dans laquelle le larvicide est choisi parmi une liste comprenant : le 4-hydroxybenzoate de méthyle, le téméphos, le diflubenzuron, le novaluron, le pyriproxyfène, le diflubenzuron, le méthoprène ou des mélanges de ceux-ci.

10. Particule selon les revendications précédentes comprenant également des vitamines, des hydratants, des cosmétiques, des huiles essentielles ou des mélanges de ceux-ci.

11. Composition comprenant les particules décrites dans les revendications précédentes.

12. Composition selon la revendication précédente destinée à être utilisée en tant que médicament, en particulier pour le traitement d'infections de la peau, de dermatite, de blessures, d'ulcères, notamment du pied diabétique.

13. Composition selon la revendication précédente comprenant également un adoucissant, un dispersant, un solvant, ou des mélanges de ceux-ci.

14. Utilisation non thérapeutique de particules selon la description des revendications 1-10 ou de la composition telle que décrite dans les revendications 11-13 en tant que fonctionnalisateur de divers matériaux et amplificateur de l'effet du composé actif au fil du temps.

15. Articles comprenant les particules et/ou compositions décrites dans les revendications précédentes ; en particulier dans lequel les articles sont : des vêtements, des chaussures, des couvre-chefs, des mortiers, des polymères, de l'argile céramique, du caoutchouc, des peintures, des ciments, des produits pharmaceutiques, des vernis, du papier, de la pâte à papier, des dispositifs médicaux, des cosmétiques, des capteurs, des détergents, des feuilles stratifiées, des couches ou des serviettes hygiéniques.
